# EUROPEAN PATENT APPLICATION

(11) **EP 4 080 206 A1**
(43) Date of publication of application: **26.10.2022**
(21) Application number: 21188510.8
(22) Date of filing: 29.07.2021
(51) Int. Cl.: G01N 33/18

(54) **A DETECTION SYSTEM FOR QUANTITATIVE ASSESSMENT OF BACTERIA IN WATER**

(30) Priority: 20.04.2021 IT 202100010016
(71) Applicant: Inthebubble S.r.l., 28100 Novara (NO) (IT); Poeta, Giuseppe, 95037 San Giovanni la Punta (CT) (IT)
(72) Inventor: Poeta, Giuseppe, 95037 San Giovanni la Punta CT (IT)
(74) Representative: Perani & Partners S.p.A.

(57) **Abstract**

A detection system (1) for quantitative assessment of bacteria in water, comprising: a feed line (2) designed to be connected to a water source to receive a water sample; at least one analysis module comprising: a housing duct (3) having an inlet portion (31) connected to the feed line (2) to receive the water sample and an outlet portion (32) designed to be connected to a drain outlet (4); a filter membrane arranged in the housing duct (3) between the inlet portion (31) and the outlet portion (32), and configured to retain the bacteria in the water sample; a differential pressure gauge (5) connected to the housing duct (3) at a first detection point located upstream from the filter membrane and at a second detection point located downstream from the filter membrane, the differential gauge (5) being configured to detect a differential value representative of the pressure difference between the first and second detection points. The detection system (1) further comprises a control device (6) in signal communication with the differential gauge (5) and configured to compare the differential value that has been detected with a control differential value for quantitative assessment of the bacteria in the water sample.

## Description

### Field of the invention

The present invention relates to a detection system for quantitative assessment of bacteria in water, known as "Bacterial Detection Unit" (BDU). Such detection system may find different applications, namely in the field of swimming pools, for example, for quantitative assessment of bacteria in water of a swimming pool for children or people with disabilities.

### Background art

As a swimming pool is used during the day, although chlorine-based disinfectants are used, the bacterial count of the swimming pool water is known to be exposed to sudden increase. Such increase of the bacterial count is caused by the release of organic material by swimmers. Therefore, knowledge of the actual bacterial count is important to avoid spreading of infections and/or diseases among the users of the swimming pool.

Also, if the bacterial count in the water of a swimming pool exceeds a given critical value, the use of the swimming pool is known to be discontinued for disinfection, e.g. according to the HACCP operator control system, if any.

At present, the only method of determining the bacterial count in the water of a swimming pool is known to be based on a bacteriological analysis performed in a laboratory.

In detail, a sample of water is collected from the swimming pool using a sterile collection device. Then, the water sample is carried to a laboratory, in which number of procedures are carried out, including seeding a suitably prepared part of the sample on "culture medium".

Then, the seeded sample is distributed in plates and tubes, and incubation is carried out under suitable conditions and at a suitable temperature.

Bacterial count assessment requires an incubation period, for microorganisms to multiply until they form colonies that are visible to the naked eye. Then the bacterial count is determined by counting the colonies.

### Problem of the prior art

Nevertheless, the method of analysis of the prior art takes a long time for assessment of the bacterial count in water. It takes more than twenty-four/thirty-six hours from collection of the water sample to obtain an estimate of the bacterial count in such sample.

As a result, while the prior art analysis method allows the bacterial count of a water sample to be determined by collecting that water sample during a given day of use of the swimming pool, the time required for analysis does not allow the bacterial count data to be readily obtained during the same day of use.

Therefore, the assessment time of the prior art analysis method is not acceptable, especially in case of swimming pools used by very young children or by users with disabilities.

Disadvantageously, with the analysis method of the prior art the use of the swimming pool cannot be timely discontinued during the same day of use in which the water sample was taken.

In other words, the use of the analysis method of the prior art cannot avoid an excessive and accidental presence of bacteria in a swimming pool and does not afford timely disinfection according to all HACCP operator control criteria to prevent the use of a swimming pool that is unsafe in terms of disease and/or infection transmission.

### Summary of the invention

Therefore, the technical purpose of the present invention is to provide a detection system for quantitative assessment of bacteria in water that can obviate the problems of the prior art.

In particular, an object of the present invention is to provide a detection system that can effectively assess the bacterial count in a water sample, a short time after collection of such water sample.

Also, an object of the present invention is to provide a swimming pool that comprises the detection system and a method of assessing the bacterial count in water using the detection system.

The aforementioned technical purpose and the specified objects are substantially fulfilled by a detection system that incorporates the technical features as set forth in one or more of the annexed claims.

### Advantages of the Invention

The detection system as described herein affords, for example, effective assessment of the bacterial count in a water sample collected from a swimming pool and allows timely actions to be taken if the bacterial count in the swimming pool exceeds a critical bacterial count value, during the same day of use in which the water sample was taken.

### BRIEF DESCRIPTION OF THE DRAWINGS

Further features and advantages of the present invention will be more readily apparent upon reading of the illustrative, non-limiting description of a preferred non-exclusive embodiment of a detection system for quantitative assessment of bacteria in water as shown in the accompanying drawings, in which:

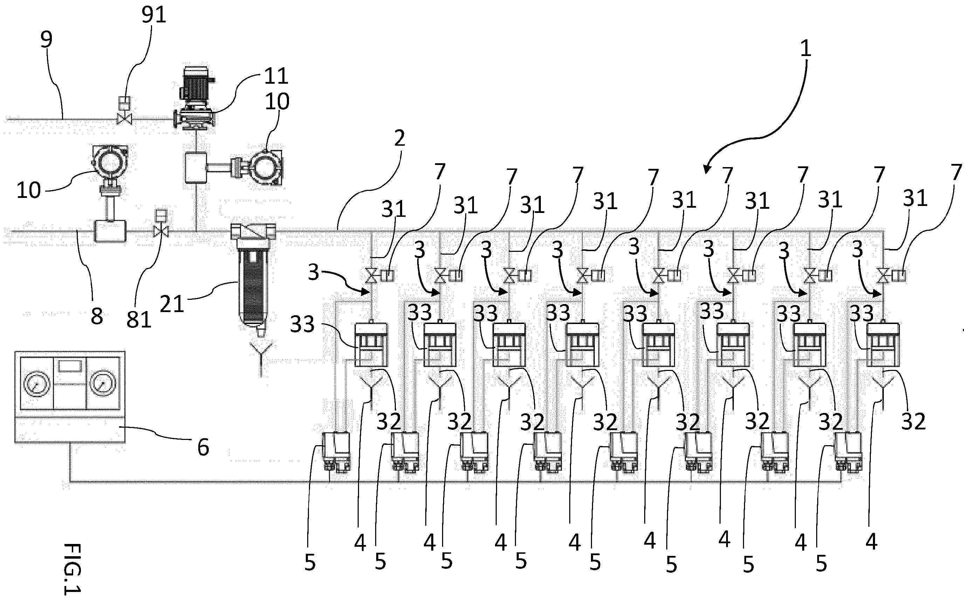

- Figure 1 is a schematic diagram of the detection system of the present invention.

### DETAILED DESCRIPTION

Particularly referring to the accompanying drawings, numeral 1 designates a detection system for quantitative assessment of bacteria in water. For example, the detection system 1 can be used for quantitative assessment of bacteria in the water of a swimming pool for children and/or people with disabilities.

The detection system 1 comprises a feed line 2 designed to be connected to a water source to receive a water sample. For example, the feed line 2 is designed to be connected to a swimming pool (not shown in the annexed figures).

In addition, the detection system 1 comprises at least one analysis module. The analysis module comprises a housing duct 3 having an inlet portion 31 and an outlet portion 32.

The inlet portion 31 of the housing duct 3 is connected to the feed line 2 to receive the water sample. On the other hand, the outlet portion 32 of the housing duct 3 can be connected to a drain outlet 4. The inlet portion 31 and the outlet portion 32 of the housing duct 3 are in fluid communication with each other.

In addition, the analysis module comprises a filter membrane arranged in the housing duct 3 between the inlet portion 31 and the outlet portion 32. For example, the housing duct 3 comprises a support 33 interposed between the inlet portion 31 and the outlet portion 32. This support 33 comprises a housing plate (not shown) for the filter membrane. This housing plate is in fluid communication with the inlet portion 31 and the outlet portion 32 of the housing duct 3. The filter membrane is housed in such housing plate of the support 33.

The filter membrane is configured to retain the bacteria in the water sample. Preferably, the filter membrane is made of cellulose and has a plurality of pores whose size is in the micron range, and is preferably 0.45 micron. In other words, the bacteria in the water sample, received by the housing duct 3, are retained by the filter membrane. The filter membrane retains bacteria larger than its pores.

Therefore, the water sample, received by the inlet portion 31 of the housing duct 3, flows from such inlet portion 31 to the outlet portion 32 of the housing duct 3 through the filter membrane. Thus, the bacteria of the water sample are retained by the filter membrane. In other words, the water sample has bacteria in it as it flows into the inlet portion 31 of the housing duct 3, and is substantially free of bacteria as it flows into the outlet portion 32 of the housing duct 3.

The analysis module further comprises a differential pressure gauge 5 connected to the housing duct 3 at a first detection point, upstream from the filter membrane, and at a second detection point, downstream from the filter membrane. For example, the first detection point is located at the inlet portion 31 of the housing duct 3 and the second detection point is located at the outlet portion 32 of the housing duct 3.

The differential gauge 5 is configured to detect a differential value representative of the pressure difference between the first and second detection points. In other words, the differential gauge 5 is configured to detect a differential value representative of the pressure difference across the filter membrane.

It should be noted that the pressure difference across the filter membrane, arranged in the housing duct 3, is representative of the bacterial count in the water sample. That is, the higher the bacterial count in the water sample that is being analyzed, the greater the amount of bacteria that accumulate on the filter membrane. The greater the amount of bacteria accumulated on the filter membrane, the greater the pressure difference across the filter membrane. Therefore, the filter membrane acts as a hydraulic resistance, whose resistance value increases as the bacteria trapped by the filter membrane increases.

The detection system 1 further comprises a control device 6 in signal communication with the differential gauge 5. The control device 6 is configured to compare the differential value that has been detected with a control differential value for quantitative assessment of the bacteria in the water sample. That is, since the detected differential value is proportional to the bacterial count in the water sample that is being analyzed, a comparison of this differential value with a control value can determine whether the bacterial count in the water sample is higher than a critical bacterial count value.

Also, since no bacteriological analysis of the collected water sample is required, there is no need to wait for a bacterial proliferation time before obtaining indications on the bacterial count of the water sample.

As a result, if the water sample is collected from a swimming pool, the analysis of such water sample using the detection system 1 affords quick and timely checks as to whether the bacterial count in the swimming pool water requires the use of the swimming pool to be discontinued and disinfection to be carried out according to all HACCP operator control criteria. Advantageously, the risks of infection and/or disease transmission among the users of the swimming pool are reduced.

It should be noted that the filter membrane arranged in the housing duct 3 can be replaced. In other words, the filter membrane can be removed from the housing duct 3. That is, once the filter membrane has been used to analyze a given water sample, it must be replaced for analysis of a different water sample.

Preferably, the analysis module comprises a weight measuring cell connected to the housing duct 3 and in signal communication with the control device 6. This weight measuring cell is configured to detect a weight value representative of the weight of the filter membrane. For example, this weight measuring cell detects the weight of the filter membrane when the water sample has passed through the filter membrane.

Still preferably, the control device 6 is configured to compare the weight value detected by the weight measuring cell with a control weight value of the filter membrane for quantitative assessment of the bacteria in the water sample. For example, the control weight value corresponds to the weight value of the filter membrane before its use in the detection system 1, i.e. before the passage of the water sample through the filter membrane.

In other words, the control device 6 is configured to quantitatively assess the bacteria in the water sample based on the comparison between the differential value detected by the differential gauge 5 and the control differential value and based on the comparison between the weight value detected by the weight measuring cell and control weight value.

According to a preferred embodiment of the invention, the housing duct 3 has an inner surface connecting the inlet portion 31 and the outlet portion 32. This inner surface defines a cross-section of the housing duct 3. The filter membrane is arranged in the housing duct 3 to span the cross section of the housing duct 3. Thus, the water sample flowing in the housing duct 3 passes through the filter membrane, allowing said filter membrane to trap the bacteria in the water sample.

Still according to the preferred embodiment of the invention, the feed line 2 comprises a filter 21 arranged upstream from the inlet portion 31 of the housing duct 3. This filter 21 is configured to retain any particles in the water sample whose size is greater than 1 micron. However, it should be noted that the bacterial species are not retained by the filter 21 and thus reach the housing duct 3. Using the filter 21, substantially only bacteria accumulate on the filtering membrane, and particles larger than those of bacteria are prevented from altering the pressure difference across the filter membrane, and from not making it only representative of the bacterial count in the water sample.

According to the preferred embodiment of the invention, the detection system 1 comprises a first analysis module and a second analysis module. It should be noted that the first and second analysis modules correspond to the analysis module as described herein. In other words, each of the first and second analysis modules comprise a respective housing duct 3, a respective filter membrane placed in the housing duct 3 and a respective differential gauge 5 in signal communication with the control device 6. Preferably, the first and second analysis modules comprise a respective weight measuring cell. It should be further noted that the first and second analysis modules are connected to the feed line 2 and to the drain outlet 4. In other words, the first and second analysis modules are arranged fluid-dynamically parallel to each other.

The first and second analysis modules are configured to receive first and second water samples, respectively. As more clearly explained hereinbelow, the first water sample corresponds to a control water sample. For example, this first water sample corresponds to a water sample whose bacterial count is known. As a further example, the first water sample comes from a water supply network. On the other hand, the second water sample corresponds to a water sample to be analyzed, i.e. a water sample whose bacterial count has to be assessed. For example, the second water sample comes from a swimming pool.

Still according to the preferred embodiment of the invention, the control device 6 is configured to generate the control differential value based on the differential pressure value detected by the differential gauge 5 of the first analysis module. In other words, the control device 6 determines the control differential value based on the differential pressure value detected when the first water sample flows through the filter membrane arranged in the housing duct 3 of the first analysis module. It should also be noted that the control device 6 is configured to store the generated control value.

Still according to the preferred embodiment of the invention, the control device 6 is configured to compare the differential value detected by the differential gauge 5 of the second analysis module with the control differential value to quantitatively assess the bacteria of the second water sample. In other words, upon comparison of the differential value detected after the passage of the second water sample through the filter membrane of the second analysis module and the control differential value, one can determine the bacteria count of the second water sample and, in particular, whether the bacterial count in the second water sample is higher than a critical bacterial count value.

According to a preferred aspect, each of the housing ducts 3 of the first and second analysis modules comprises a control valve 7. For example, each control valve 7 corresponds to a solenoid valve in signal communication with the control device 6.

Each control valve 7 is adapted to be switched between an open configuration, in which the feed line 2 and its respective housing duct 3 are in fluid communication with each other, and a closed configuration, in which such control valve 7 hydraulically seals the feed line 2 and its respective housing duct 3 from each other. Preferably, each control valve 7 is adapted to be switched between the open configuration and the closed configuration by the control device 6.

In order to allow the first water sample to flow from the feed line 2 to the housing duct 3 of the first analysis module, the control valve 7 of the first analysis module is switched to and kept in the open configuration, while the control valve 7 of the second analysis module is switched to and kept in the closed configuration.

Conversely, in order to allow the second water sample to flow from the feed line 2 to the housing duct 3 of the second analysis module, the control valve 7 of the first analysis module is switched to and kept in the closed configuration, while the control valve 7 of the second analysis module is switched to and kept in the open configuration.

Preferably, the detection system 1 comprises, in addition to the first and second analysis modules, a plurality of additional analysis modules arranged fluid-dynamically parallel to the first and second analysis modules. Each additional analysis module is configured to receive a respective additional water sample. Still preferably, each of the housing ducts 3 of the additional analysis modules comprises a respective control valve 7 corresponding to the control valves 7 of the first and second analysis modules.

Also, still preferably, the control device 6 is configured to compare the differential value detected by the differential gauge 5 of each additional analysis module with the control differential value to quantitatively assess the bacteria of the additional water sample received by the respective additional analysis module.

A detection system 1 comprising a plurality of additional analysis modules can detect, for example, the bacterial count in a swimming pool at given times of a day of use of such swimming pool, to check whether there is a progressive increase in the bacterial count during that day of use. As a further example, the bacterial count may be assessed every hour of use of the swimming pool.

According to the preferred embodiment of the invention, the detection system 1 comprises a first sampling line 8 and a second sampling line 9 which can be connected to a first water source and a second water source respectively. For example, the first water source corresponds to a water supply network, and the second water source corresponds to a swimming pool. In other words, the first sampling line 8 is configured to receive water from the water supply network, and the second sampling line 9 is configured to receive water from the swimming pool.

It should be noted that, according to what has been stated hereinbefore, the water from the water supply network is used to determine the control differential value. In other words, the first water sample is taken from the water supply network. Conversely, the water from the swimming pool corresponds to the water to be analyzed. In other words, the second water sample and the additional water samples are taken from the swimming pool.

According to a preferred aspect, the first sampling line 8 and the second sampling line 9 comprise a first selection valve 81 and a second selection valve 91. Preferably, both the first selection valve 81 and the second control valve 91 correspond to a solenoid valve in signal communication with the control device. Each selection valve 81, 91 is designed to be switched between an open configuration, in which its respective sampling line 8,9 is in fluid communication with the feed line 2, and a closed configuration, in which its respective sampling line 8,9 is hydraulically sealed from the feed line 2. Preferably, each selection valve 81,91 is designed to be switched between its respective open configuration and its respective closed configuration by the control device 6, based on the various planning sequences.

It should be noted that when the first water sample is to be introduced into the first analysis module, the selection valve 81 of the first sampling line 8 is switched to and kept in the open configuration, while the selection valve 91 of the second sampling line is switched to and kept in the closed configuration.

Conversely, when the second water sample is to be introduced into the second analysis module or a respective additional water sample is to be introduced into a respective additional analysis module, the selection valve 91 of the second sampling line 9 is switched to and kept in the open configuration, while the selection valve 81 of the first sampling line 8 is switched to and kept in the closed configuration.

Still according to the preferred embodiment of the invention, each sampling line 8, 9 comprises a flow-rate regulator 10. Still preferably, the second sampling line 9 comprises a water circulator 11. Flow-rate regulators 10 and the water circulator 11 are well-known to the skilled person and will not be further described herein.

The present invention also relates to a swimming pool.

This swimming pool comprises a tank (not shown) configured to contain water. In addition, this swimming pool comprises the detection system 1 as described herein.

The feed line 2 of the detection system 1 is connected to the swimming pool tank to receive water from the tank. For example, the feed line 2 is connected to a drain of the swimming pool tank.

Finally, the present invention relates to a method of quantitative assessment of bacteria in water. This method of quantitative assessment of bacteria in water uses the detection system 1 as described herein.

This method comprises the step of connecting the detection system 1 to a water source to introduce a water sample from such water source into the feed line 2. For example, the detection system 1 is connected to a swimming pool.

Then, the method comprises the step of detecting by differential pressure gauge 5 of the at least one analysis module, a differential pressure value and sending such differential pressure value to the control device 6.

The method further comprises the step of comparing, by the control device 6, the detected differential pressure value with a control differential value to assess the bacteria of the water sample.

Preferably, the step of comparing the detected differential pressure value with a control differential value for assessing the bacterial count of the water sample comprises the step of defining a critical bacterial count threshold and storing such critical bacterial count threshold in the control device 6.

Once the critical bacterial count threshold has been defined, the step of comparing the detected differential pressure value with a control differential value for assessing the bacterial count of the water sample comprises the step of sending an alarm signal when the detected differential pressure value is higher than the control differential value by a value that is greater than or equal to the critical bacterial count threshold, i.e. when the detected differential pressure value exceeds a critical bacterial count value.

Preferably, the alarm signal is sent via the control device 6 to an external device such as, for example, a smartphone.

Still preferably, the method comprises the step of detecting, by the weight measuring cell of the at least one analysis module, a weight value of the filter membrane and sending said weight value to the control device 6.

The method further comprises the step of comparing, by the control device 6, the weight value detected by the weight measuring cell with a control weight value for the filter membrane to assess the bacteria count of the water sample. In other words, the bacteria count of the water sample is determined by comparing the differential pressure value detected by the differential gauge 5 with the control differential value and by comparing the weight value detected by the weight measuring cell with the control weight value.

## Claims

1. A detection system (1) for quantitative assessment of bacteria in water, comprising:
- a feed line (2) designed to be connected to a water source to receive a water sample;
- at least one analysis module comprising:
- a housing duct (3) having an inlet portion (31) connected to the feed line (2) for receiving the water sample, and an outlet portion (32) designed to be connected to a drain outlet (4);
- a filter membrane arranged in the housing duct (3) between the inlet portion (31) and the outlet portion (32), said filter membrane being configured to retain the bacteria in the water sample;
- a differential pressure gauge (5) connected to the housing duct (3) at a first detection point, upstream from the filter membrane, and at a second detection point, downstream from the filter membrane, said differential gauge (5) being configured to detect a differential value representative of the pressure difference between the first and second detection points;
- a control device (6) in signal communication with the differential gauge (5) said control device (6) being configured to compare the detected differential value with a control differential value for quantitative assessment of bacteria in the water sample.

2. A detection system (1) as claimed in claim 1, wherein:
- the analysis module comprises a weight measuring cell connected to the housing duct (3) and in signal communication with the control device (6), said weight measuring cell being configured to detect a weight value representative of the weight of the filter membrane;
- the control device (6) is configured to compare the weight value detected by the weight measuring cell with a control weight value of the filter membrane for quantitative assessment of the bacteria in the water sample.

3. A detection system (1) as claimed in claim 1 or 2, wherein:
- the housing duct (3) has an inner surface connecting the inlet portion (31) and the outlet portion (32), said inner surface defining a cross section of said housing duct (3);
- the filter membrane is arranged in the housing duct (3) to span the cross section of said housing duct (3).

4. A detection system (1) as claimed in any of claims 1 to 3, wherein the filter membrane is made of cellulose and has a plurality of pores whose size is in the micron range, and is preferably 0.45 micron.

5. A detection system (1) as claimed in any of claims 1 to 4, wherein the feed line (2) comprises a filter (21) arranged upstream from the inlet portion (31) of the housing duct (3).

6. A detection system (1) as claimed in any of claims 1 to 5, comprising a first analysis module and a second analysis module which are configured to receive a first water sample and a second water sample respectively, the control device (6) being configured to generate the control differential value based on the differential pressure value detected by the differential gauge (5) of the first analysis module and being configured to compare the differential value detected by the differential gauge (5) of the second analysis module with the control differential value for quantitatively assessing the bacteria of the second water sample.

7. A detection system (1) as claimed in claim 6, wherein:
- the housing ducts (3) of the first and second analysis modules each comprise a control valve (7), each control valve (7) being adapted to be switched between an open configuration, in which the feed line (2) and its respective housing duct (3) are in fluid communication with each other, and a closed configuration, in which said control valve (7) hydraulically seals the feed line (2) and its respective housing duct (3) from each other.

8. A detection system (1) as claimed in any of claims 1 to 7, comprising:
- a first sampling line (8) and a second sampling line (9) designed to be connected to a first water source and a second water source respectively;
- the first sampling line (8) and the second sampling line (9) comprise a first selection valve (81) and a second selection valve (91) respectively, each selection valve (81, 91) being designed to be switched between an open configuration, in which its respective sampling line (8,9) is in fluid communication with the feed line (2), and a closed configuration, in which its respective sampling line (8,9) is hydraulically sealed from the feed line (2).

9. A swimming pool comprising:
- a tank configured to contain water;
- a detection system (1) as claimed in any of claims 1 to 8, the feed line (2) of the detection system (1) being connected to the tank to receive water from the tank.

10. A method for quantitative assessment of bacteria in water, said method comprising the steps of:
- providing a detection system (1) as claimed in any of claims 1 to 8,
- connecting said detection system (1) to a water source to introduce a water sample from said water source into the feed line (2);
- detecting, by differential pressure gauge (5) of the at least one analysis module, a differential pressure value and sending said differential pressure value to the control device (6);
- comparing, by the control device (6), the detected differential pressure value with a control differential value to assess the bacteria of the water sample.
